# EUROPÄISCHE PATENTSCHRIFT

(11) **EP 2 844 632 B1**
(45) Veröffentlichungstag und Bekanntmachung des Hinweises auf die Patenterteilung: **30.03.2016**
(21) Anmeldenummer: 13719833.9
(22) Anmeldetag: 29.04.2013
(51) Int. Cl.: C07C 51/41, C07C 53/10, C07C 55/06, C07C 59/08

(54) **VERFAHREN ZUR UMSETZUNG VON GLYCERIN ZU ORGANISCHEN SALZEN**
METHOD FOR REACTING GLYCERIN TO FORM ORGANIC SALTS
PROCÉDÉ DE TRANSFORMATION DE GLYCÉRINE EN SELS ORGANIQUES

(30) Priorität: 03.05.2012 LU 91993
(43) Veröffentlichungstag der Anmeldung: 11.03.2015
(73) Patentinhaber: D.01 P.A.C. Holding, L-3980 Wickrange (LU)
(72) Erfinder: ROLLINGER, Guy, L-3980 Wickrange (LU); KEMPF, Armin, 55234 Wendelsheim (DE); MATERNOVA, Jindriska, L-7733 Colmar-Berg (LU)
(74) Vertreter: Office Freylinger
(86) Internationale Anmeldenummer: PCT/EP2013/058875
(87) Internationale Veröffentlichungsnummer: WO 2013/164301

(56) Entgegenhaltungen:
- JP-A- 2005 200 340
- US-B2- 7 829 740
- HISANORI KISHIDA ET AL: "Conversion of Glycerin into Lactic Acid by Alkaline Hydrothermal Reaction", CHEMISTRY LETTERS, CHEMICAL SOCIETY OF JAPAN, JP, Bd. 34, Nr. 11, 1. November 2005 (2005-11-01), Seiten 1560-1561, XP008107335, ISSN: 0366-7022, DOI: 10.1246/CL.2005.1560 [gefunden am 2005-10-15]

## Beschreibung

### Technisches Gebiet

Die vorliegende Erfindung betrifft allgemein ein Verfahren zur Umsetzung von Glycerin in stark konzentrierten, flüssigen, wässrigen Lösungen von Metallhydroxiden.

### Stand der Technik

Glycerin (IUPAC Glycerol, auch Glyzerin) ist der Trivialname und die gebräuchliche Bezeichnung von Propan-1,2,3-triol. Die Herstellung von Glycerin kann petrochemisch aus Propen mit den Zwischenprodukten Allylchlorid und Epichlorhydrin oder chemisch als Nebenprodukt bei der Verseifung von natürlichen Fetten und Ölen zur Gewinnung von Seifen geschehen. Inzwischen werden große Mengen Glycerin als Nebenprodukt der Biodieselherstellung erzeugt. Dies geschieht durch eine Umesterung von meist pflanzlichen Ölen mit Methanol. Ein Fettmolekül (Triacylglycerid) wird mit drei Methanolmolekülen zu Glycerin und drei Fettsäuremethylestern (FAME) umgesetzt.

Bei der Umesterung fallen prozessbedingt 100 kg Glycerin pro Tonne Biodiesel an. Bisher stehen zu wenige wirtschaftliche Verwertungswege für dieses Glycerin offen. Die traditionellen Anwendungsgebiete für Glycerin in der Kosmetik,- Nahrungs-, Genussmittel- und Pharmaindustrie sind weitgehend ausgeschöpft.

Überschüssige Glycerinmengen aus der Biodieselherstellung werden daher teilweise mit dem Abwasser entsorgt bzw. zur Energieerzeugung verbrannt.

Nutzungsalternativen sind beispielsweise die Erzeugung von Biogas, chemische Umsetzung zu Kraft- und Schmierstoffen oder die Verwendung als Futtermittelzusatz.

Glycerin eignet sich wegen seiner guten chemischen Reaktivität auch als Grundstoff für die Herstellung von Chemikalien, die sonst aus Erdöl gewonnen werden. Neue glycerinbasierte Produktionsverfahren, beispielsweise für Epichlorhydrin oder Propylenglykol sind in Erprobung und in zahlreichen Forschungsvorhaben werden Verfahren erarbeitet, um neue Einsatzgebiete für Glycerin als Rohstoff zu erschließen.

Es gibt jedoch kaum ausgereifte Verwertungstechnologien für Rohglycerin.

US 7,829,740 B2 beschreibt ein Verfahren zur Herstellung von Lactat aus Glycerin, indem das Glycerin einer hydrothermalen Reaktion unter alkalischen Bedingungen ausgesetzt wird.

### Aufgabe der Erfindung

Eine Aufgabe der vorliegenden Erfindung ist es, Glycerin und bevorzugt Glycerin aus der Biodieselherstellung in ein wirtschaftlich wertvolles Produkt umzuwandeln.

Diese Aufgabe wird durch ein Verfahren nach Anspruch 1 gelöst.

### Allgemeine Beschreibung der Erfindung

Insbesondere wird diese Aufgabe gelöst durch ein Verfahren zur Umsetzung von Glycerin wobei das Glycerin in einer wässrigen, flüssigen Lösung von Me-Hydroxid, wobei die Konzentration des Me-Hydroxids größer als die Sättigungskonzentration bei Raumtemperatur ist, bei Temperaturen über 150°C umgesetzt wird, wobei Me ausgewählt ist aus der Gruppe bestehend aus Alkali- und Erdalkalimetallen und deren Mischungen und mindestens ein Salz ausgewählt aus der Gruppe bestehend aus Formiat, Acetat, Propionat, Oxalat, Lactat, Butyrat, Valerat, Citrat, Adipat, Succinat, Malat und Carbonat sowie deren Mischungen und Wasserstoffgas (H₂) oder Methan (CH₄) sowie deren Mischungen entstehen.

Das Verfahren wird demnach in einer stark konzentrierten, wässrigen Lösung von Me-Hydroxid, wobei die Konzentration des Me-Hydroxids größer als die Sättigungskonzentration bei Raumtemperatur ist, bei Temperaturen im Liquidusbereich des Systems zwischen Schmelz- und Siedepunkt bevorzugt nahe am Siedepunkt durchgeführt.

Bevorzugt wird das Verfahren bei einem Druck von 800 bis 1500 hPa, bevorzugt zwischen 950 und 1050 hPa und besonders bevorzugt bei atmosphärischem Druck ausgeführt.

Bevorzugt wird als Me-Hydroxid NaOH oder KOH sowie deren Mischungen eingesetzt.

Nach einer bevorzugten Ausführung ist die Konzentration von Me-Hydroxid größer als 54 Gew %, bevorzugt größer als 60 Gew %, besonders bevorzugt größer als 70 Gew % und ganz besonders bevorzugt größer als 80 Gw % und insbesondere größer als 85 Gew %.

Die Reaktion wird bevorzugt bei mehr als 150°C, besonders bevorzugt bei mehr als 160°C und ganz besonders bevorzugt bei mehr als 180°C und insbesondere bei mehr als 200°C ausgeführt.

Im Gegensatz zu dem in dem US Patent US 7,829,740 B2 beschriebenen Verfahren, kann im erfindungsgemäßen Verfahren ohne hohen Druck gearbeitet werden, da sich bei der hohen Konzentration von Me-Hydroxid die Siedetemperatur der Lösung sehr stark erhöht und deutlich höher als 100°C ist. Außerdem erfolgt zumindest teilweise Abtrennung des erzeugten Salzes in einem direkten Schritt mit der Reaktion ohne zusätzliche Trennschritte. Dadurch ist das Verfahren wesentlich einfacher und sicherer auszuführen als das Verfahren aus dem US Patent US 7,829,740 B2. Wegen der hohen Hydroxid-Konzentration, ist es möglich, bei hohen Temperaturen ohne Überdruck zu arbeiten, weil die Siedetemperatur einer stark konzentrierten wässrigen Lösung von Me-Hydroxid, wesentlich über der Siedetemperatur von Wasser liegt.

Die Reaktion wird bevorzugt bei Temperaturen am Siedepunkt der Hydroxid-Lösung ausgeführt, wobei der Siedepunkt mit zunehmender Konzentration der Hydroxid-Lösung steigt. Um das Wasser am Verlassen des Reaktionsgefäßes zu hindern, wird bevorzugt ein Rückflusskühler eingesetzt. An den Kühlflächen können sich die während der Reaktion gebildeten Dämpfe kondensieren und der Reaktionsmischung wieder zugeführt werden. Als Kühlmittel wird im Rückflusskühler z.B. fließendes Wasser eingesetzt.

Unter "Temperaturen am Siedepunkt" versteht man im Zusammenhang mit der Erfindung einen Temperaturbereich innerhalb von 15°C, bevorzugt innerhalb von 10°C, ganz besonders bevorzugt innerhalb von 7.5°C und insbesondere innerhalb von 5°C um den Siedepunkt der Me-Hydroxid-Lösung.

Liegt z.B. der Siedepunkt der verwendeten Natriumhydroxid-Lösung bei 150°C, versteht man im Zusammenhang mit der Erfindung einen Temperaturbereich von 135°C bis 165°C. Die Siedetemperatur verschiebt sich zudem in nicht vorhersagbarer Weise beim Übergang vom binären Stoffsystem Me-Hydroxid-H₂O zum Multikomponentensystem durch Zusatz von Glycerin und durch die nach dem Zusatz von Glycerin entstehenden Reaktionsprodukte.

Gemäss einer bevorzugten Ausführung, wird das Verfahren mit NaOH im Konzentrationsbereich 60-80% bei Temperaturen unterhalb von 180°C ausgeführt. Bei diesen Temperaturen wird hauptsächlich Lactat erzeugt.

Überraschenderweise wurde festgestellt, dass bei diesen Bedingungen die Lactatausbeute mit über 97% sehr viel höher liegt, als außerhalb dieses Bereiches. Versuche im Zusammenhang mit der Erfindung haben ergeben, dass z.B. bei 83% NaOH und 222°C nur noch 10.5 % Lactat entsteht, während bei 67% / 175°C der Lactatgehalt des Endproduktes bei rund 98% liegt.

Gemäss einer zweiten bevorzugten Ausführung, wird das Verfahren bei Temperaturen zwischen 220-260°C, vorzugsweise bei 235°C ausgeführt und es wird hauptsächlich Acetat, Propionat oder Oxalat sowie deren Mischungen erzeugt. Bei atmosphärischem Druck und bei Konzentrationen von über 85% NaOH bevorzugt von über 93% NaOH und Temperaturen von über 220°C, bevorzugt über 260°C wird hauptsächlich Acetat oder Propionat sowie deren Mischungen erzeugt.

Überraschenderweise wurde festgestellt, dass bei diesen Temperaturen hauptsächlich Acetat, Propionat und Oxalat entsteht und kein Lactat. Bei niedrigeren Temperaturen entsteht Lactat. Versuche im Zusammenhang mit der Erfindung haben ergeben, dass z.B. bei 235°C, 35 Gew% Acetat, 30 Gew% Propionat und 25 Gew% Oxalat entstehen und bei Temperaturen von 260°C 52 Gew% Acetat, 42 Gew% Propionat entsteht.

Gemäss einer weiteren bevorzugten Ausführung, wird die wässerige Lösung von Me-Hydroxid unter ständigem Umrühren bei Temperaturen nahe am Siedepunkt gehalten und das Glycerin in die Me-Hydroxid Lösung zudosiert.

Das Glycerin wird bevorzugt vorgewärmt, bevor es in die Me-Hydroxid Lösung zu dosiert wird.

Als Me-Hydroxid wird besonders bevorzugt NaOH verwendet.

Das Glycerin stammt bevorzugt aus der Herstellung von Biodiesel.

Das Verfahren kann entweder kontinuierlich sein oder aber ein Batch-Verfahren.

Gemäss einer bevorzugten Ausführung wird das durch die Umsetzung von Glycerin entstehende Wasser ganz oder zum Teil entzogen.

### Ausführlichen Beschreibung der Erfindung an Hand von Beispielen

Weitere Einzelheiten und Vorteile der Erfindung können der nachfolgenden ausführlichen Beschreibung möglicher Ausführungsformen der Erfindung anhand der Beispiele entnommen werden.

Die Umsetzung von Glycerin mit hochkonzentrierten wässrigen NaOH-Lösungen im Konzentrationsbereich ≥ 53 Gew% NaOH wurde bei Temperaturen nahe am Siedepunkt der NaOH-Lösungen getestet. Als Glycerin wird bevorzugt Glycerin aus der Biodieselherstellung eingesetzt. Bei den Versuchen wurde jedoch der Einfachheit halber, reines Glycerin sowie Glycerin mit ähnlichen Wassergehalten wie Rohglycerin aus der Biodieselherstellung eingesetzt.

Bei den Versuchen umfasste das Verfahren folgende Schritte:
- Feste NaOH-Plätzchen werden in verschiedenen Verhältnissen mit H₂O gemischt und unter Rückfluss erhitzt.
- Die maximale Betriebstemperatur ergibt sich aus dem Siedepunkt bei atmosphärischen Druck des Gemisches NaOH - H₂O und liegt konzentrationsabhängig bei NaOH Konzentration > 50 Gew% im Temperaturbereich von ca. 140 °C bis über 300°C.
- Glycerin wird unter die Oberfläche der gerührten NaOH-Lösung zudosiert, wonach eine schnelle exotherme Reaktion erfolgt. Bei Batch-Verfahren werden Glycerin, Wasser und NaOH gemischt und dann erhitzt.
- Die einzigen gasförmigen Reaktionsprodukte sind H₂ und CH₄ mit H₂ -Anteilen von über 90 Vol-% .
- Als Reaktionsprodukte entstehen Soda (Na₂CO₃) und organische Natriumsalze aus der Gruppe bestehend aus Formiat, Acetat, Propionat, Oxalat, Lactat, Butyrat, Valerat, Citrat, Adipat, Succinat und Malat.
- Die Reaktion kann gezielt so gesteuert werden, dass bestimmte Salze bevorzugt entstehen.

### Vergleichsbeispiel: Chargenweises bzw. Batch-Verfahren bei Atmosphärendruck und NaOH kleiner als 54 % (Labor):

Durch Zugabe von ca. 200 g NaOH in ca. 150 ml H₂0 wurde eine bei Raumtemperatur (23 °C) gesättigte Lösung mit Bodensatz hergestellt. Die gesättigte Lösung mit nominal 47 Gew% H₂O / 53 Gew% NaOH (laut Literatur-Phasendiagramm) wurde vom Bodensatz in einen Mehrhalsglaskolben abgeschüttet. Es wurden 2 ml reines Glycerin (99,6%) hinzugegeben und die Mischung unter Rühren langsam erhitzt. Vor dem Aufheizen wurde der Gasraum zunächst evakuiert und dann mit Stickstoff gespült. Über einen Y-Abzweig mit Absperrhahn konnte sich entwickelndes Gas über ein aufgesetztes Gaskühlrohr zu einem Gaszähler geleitet werden.

Die Temperatur der Reaktionsmischung wurde über ein eingetauchtes Thermoelement gemessen. Bei 144 °C begann eine starke Blasenentwicklung, die auf das Erreichen des Siedepunktes (Literaturwert 147 °C) zurückgeführt werden muss, da mit dem angeschlossenen Gaszähler keine Volumenzunahme erfasst werden konnte. Die Temperatur wurde auf knapp unterhalb des Siedepunktes gesenkt und dort über mehr als 1 Stunde gehalten. Mit einer Lupe konnten vereinzelte Gasbläschen beobachtet werden, wobei nicht unterschieden werden kann, ob es sich um Gas aus einer Stoffumsetzung oder um Wasserdampfblasen handelt.

Die ionenchromatographische Analyse der Reaktionsmischung ergab keinerlei Reaktionsprodukte. Eine Gasanalyse war mangels Menge nicht möglich.

### Beispiel 1: Chargenweises bzw. Batch-Verfahren bei Atmosphärendruck (Pilot):

25 kg feste NaOH, 5 kg Wasser und 3.3 kg Glycerin (75% NaOH / 15% H₂O/ 10% Glycerin) wurden in einen Rührreaktor geladen und bei Raumtemperatur unter Inertgas (N₂) vermischt. Bei Raumtemperatur löst sich dabei nur ein Teil des NaOH ( max. etwa 5 kg bezogen auf reines Wasser) und es entstand ein klebriger Brei. Diese inhomogene Mischung wurde langsam mit ca. 2°C pro Minute hochgeheizt, wobei sich das NaOH zunehmend auflöste. Der Siedepunkt der Mischung erhöht sich mit zunehmender Auflösung des NaOH. Es wurde darauf geachtet, dass die Mischung nicht zum Sieden kam.

Eine homogene flüssige Mischphase wurde erst bei ca. 185 °C erreicht. Zuvor wurden schon bei ca. 160 °C die Bildung von ersten Gasblasen (H₂) beobachtet, die den Beginn der gewünschten Reaktion markierten. Die Gasentwicklung nahm mit fortschreitender Temperaturerhöhung zu. Die Temperatur wurde stetig bis auf 218 °C erhöht. Dies war systembedingt die Maximaltemperatur bei Normaldruck, weil die Mischung dort heftig siedete.

Die Reaktionsmischung wurde weitere 4 Stunden unter nur noch leichtem Sieden bei 215 °C gehalten. Das entstehende Gas wurde über ein Auslassrohr abgeführt und dabei zusammen mit dem Wasserdampf am Rückflusskühler gekühlt.

Die Zusammensetzung des Gases wurde mit einer Wärmeleitfähigkeitsmesszelle kontinuierlich gemessen und erreichte Höchstwerte von 100 Vol% Wasserstoff.

Mit fortschreitender Reaktionszeit wurde in der flüssigen Reaktionsmischung die Zunahme einer festen dispergierten Phase beobachtet. Von dieser Suspension wurde eine Probe zur späteren Analyse abgesaugt.

Die Analyse auf organische Natriumsalze erfolgte mittels Kapillarelektrophorese und ergab die Resultate in der folgenden Tabelle. Aus 3,3 kg anfangs eingesetzten Glycerins wurden 4,1 kg organische Salze erhalten, womit die Ausbeute gewichtsbezogen bei über 100% läge. In der Tabelle ist daher nur die Kohlenstoffbilanz dargestellt, d.h. das Verhältnis des in den Produkten wiedergefundenen Kohlenstoffs (C-Salze) zu dem Gesamtkohlenstoff im Input-Glycerin (C-Glycerin)

| **Natriumsalz** | **% C_{salz} /C_{glycerin}** |
|---|---|
| Formiat | 6,2 |
| Acetat | 26,0 |
| Propionat | 8,2 |
| Oxalat | 17,9 |
| Lactat | 12,9 |
| Sonstige (Butyrat, Valerat, Citrat, u.a.) | ca. 1 |

Insgesamt finden sich über 70 Gew% des Input-Kohlenstoffs in den gebildeten organischen Na-Salzen wieder, vor allem als Acetat und Oxalat. Der Rest des Input-Kohlenstoffes findet sich anorganisch gebunden als Na₂CO₃ wieder. Der Kohlenstoffanteil im Methananteil des Prozessgases wurde hier außer Acht gelassen und nicht in die Rechnungen mit einbezogen.

Die Umsetzung des Glycerins kann somit als fast vollständig angesehen werden.

### Beispiel 2: Semikontinuierliches bzw. Fed-Batch-Verfahren bei Atmosphärendruck ( Labor ):

### Beispiel 2a): bei NaOH kleiner als 50 Gew%

In einem Glaskolben wurden 15,04 g NaOH mit 34,34 g Wasser vermischt und die Mischung nach Spülung mit Stickstoff im Sandbad bis zum Siedepunkt erhitzt. Die Mischung mit nominal 30,45 % NaOH-Gehalt wurde ständig am Sieden gehalten ( gemessene Temperatur mit eingetauchtem Thermoelement 119 °C ), wobei der entstehende Wasserdampf an einem Rückflusskühler kondensiert wurde und das Wasser in die Lösung zurücktropfte.

Es wurden 0,5 ml wasserfreies reines Glycerin über eine Kapillare unter die Oberfläche der siedenden Mischung eingespritzt. Dies wurde 2 Minuten später wiederholt und 10 min später nochmals mit einer Menge von 1 ml wiederholt. Die Reaktionsmischung wurde 105 min auf Siedetemperatur gehalten.

Es konnte keinerlei Reaktionsgeschehen, z.B. in Form von Schäumen, Eintrüben o.ä., beobachtet werden.

Nach dem Abkühlen wurde die gesamte Reaktionsmischung ionenchromatographisch analysiert. Es konnte keinerlei Reaktionsprodukt festgestellt werden.

### Beispiel 2 b) bei NaOH grösser 54 Gew%

87.5 g feste NaOH-Plätzchen und 14.6 g destilliertes Wasser (entsprechend 85.7 Gew% NaOH und 14.3 Gew% H₂O) wurden in einen Mehrhalsglaskolben eingewogen. Der Kolben wurde mit N₂ inertisiert und unter Rühren zunächst bis zur vollständigen Lösung des NaOH unter Rückfluss hochgeheizt ( ca. 220 °C ). Der Ausgang des Rückflusskühlers war mit einem Gaszähler verbunden.

Die Temperatur wurde anschließend weiter langsam erhöht bis zur maximalen Betriebstemperatur, die bei atmosphärischem Druck möglich ist, d.h. bis zum Siedepunkt des Gemisches von nominal ca. 230 °C (extrapoliert aus der Literatur), im konkreten Falle bei gemessenen 243 °C (die Differenz ist vermutlich auf eine Konzentrationsverschiebung durch den im Rückflusssystem zirkulierenden Wasseranteil zurückzuführen). Die Temperaturmessung erfolgte mit einem Thermoelement, das in die Reaktionsmischung eintauchte.

Nach der Temperaturstabilisierung am Siedepunkt wurde über eine kalibrierte 2 ml Kolbenspritze und eine im Hals des Glaskolbens eingedichtete Edelstahlkapillare 0.7 g leicht vorgewärmtes reines Glycerin ( 99,6 % , ca 35 °C) ca. 1 cm unter die Oberfläche der gerührten NaOH-Lösung schlagartig zudosiert.

Auf die Glycerinzugabe folgte eine sofortige Gasentwicklung. Mittels einer gasdichten Spritze wurde ca. alle 10 Minuten eine Gasprobe durch ein Septum abgezogen und sofort mittels Gaschromatographie analysiert. Die Ergebnisse lagen bei ca. 96 Vol% H₂ und 4 Vol% CH₄.

Nach ca. 1 Stunde wurde nochmals 1.31 g Glycerin zudosiert und danach Gasproben gezogen, die ca. 98 Vol% H₂ und 2 Vol% CH₄ ergaben.

Bei der Glycerinzudosierung wurde die unmittelbare Bildung eines weißen Feststoffes beobachtet. Er bleibt beim Rühren in Dispersion und erhöht im Laufe des Versuches die Viskosität der Reaktionsmischung (erschwertes Rühren).

Der Kolbeninhalt wurde später auf Carbonat- und Organikgehalt analysiert. Es wurden Na₂CO₃, Na-Formiat, Na-Acetat, Na-Propionat und Na-Oxalat nachgewiesen, wobei methodenbedingt nicht alle Stoffe erfasst werden konnten. Eine Isolierung und quantitative Analyse der Einzelstoffe erfolgte nicht.

### Beispiel 3: Semikontinuierliches bzw. Fed-Batch-Verfahren bei Atmosphärendruck (Pilot):

20 kg Wasser wurden im Rührreaktor auf ca. 70 °C vorgeheizt, so dass fast kein Wasserdampf entstand. Über einen Füllstutzen wurde NaOH portionsweise so hinzugeschüttet und verrührt, dass die wegen der starken Lösungswärme ansteigende Temperatur weit unter dem Siedepunkt der Lösung blieb und kein Wasserdampf durch den Füllstutzen entweichen konnte. Die geringen entstandenen Mengen Wasserdampf wurden sowohl an einem Rückflusskühler kondensiert, als auch vom NaOH im Einfüllstutzen absorbiert, wobei letzterer zusätzlich mit einer dichten Plastikhülle abgedeckt war. Unter weiterem Rühren und portionsweiser Zugabe von NaOH bis zu einer Gesamtmenge von 100 kg wurde die Temperatur der Mischung sukzessive durch äußeres Beheizen des Reaktors so weit erhöht, bis das gesamte NaOH gelöst war. Die mit mehreren Eintauch-Thermoelementen gemessene Liquidustemperatur lag zwischen 180 °C und 190 °C ( Literaturwert für 83.3 Gew% NaOH = 185 °C). Nach Verschließen des Füllstutzens wurde der Reaktorinhalt unter Rückfluss bis zur Siedetemperatur hochgeheizt und dann auf dieser Temperatur bzw. bis zu 5 °C niedriger als diese Temperatur gehalten.

Nach Inertisieren mit Stickstoff wurde reines Glycerin (99.6%) auf 70 °C in einem Vorlagegefäss vorgeheizt und dann über eine Ringdüse ca. 25 cm unter der Oberfläche der gerührten Reaktionsmischung zudosiert.

In mehreren Versuchsserien wurden unter analoger Vorgehensweise die NaOH Konzentration zwischen 68 Gew% und 93 Gew%, die Reaktionstemperaturen zwischen 175 °C und 270 °C und die Glycerin-Zufuhrraten zwischen 0.5 kg/h und 25 kg/h variiert.

Sofort nach Beginn der Zudosierung setzte jedesmal eine sofortige Gasentwicklung ein mit schnell wachsendem H₂ Anteil. Volumen und Gaszusammensetzung wurden on-line erfasst bzw. analysiert mittels Gaszähler und speziellem Wärmeleitfähigkeitsdetektor.

Es wurden unterschiedliche Gasbildungsraten gemessen, was auf einen Ablauf mehrerer Folgereaktionen mit Bildung verschiedener Produkte hinwies.

Nach Reaktionszeiten zwischen ca. 30 und 90 min wurde immer ein Maximum in der Wasserstoffkonzentration erreicht, welches nie unter 97 Vol% lag und auch Werte von 100 % erreichte. Zur genaueren Analyse wurden zusätzlich Gasproben über einen Probenahmebehälter aus dem Gasstrom entnommen. Darin wurden gaschromatographisch nur geringe Anteile von Methan und Spuren von weiteren organischen Stoffen im ppm-Bereich gefunden.

Der Innenraum des Rührreaktors konnte durch ein Schauglas visuell beobachtet werden, wobei man neben der Gasbildung auch die Bildung eines festen Reaktionsproduktes beobachten konnte. Dieses wurde durch die von der Ringdüse aufsteigenden Gasbläschen flächendeckend an die Oberfläche des Reaktionsmediums flotiert und bildete dort eine lockere Schaumschicht, die sich zunehmend zu einem dickeren Kuchen verdichtete.

Nach Beendigung der Glycerinzudosierung wurde die Temperatur noch über mindestens 2 Stunden gehalten und eine Nachreaktion mit Gasentwicklung beobachtet. Wenn die Gasentwicklung fast zum Stillstand gekommen war (kleiner 1 l/min) wurde der Reaktor mit Stickstoff inertisiert, geöffnet und das Flotationsprodukt vollständig abgeschöpft.

Die späteren Analysen mittels Kapillarelektrophorese, Ionenchromatographie, FTIR, XRD, TIC, TOC, TGA ergaben ein Gemisch aus organischen Natriumsalzen (Natriumcarboxylaten) von je nach Reaktionsbedingung variablen Anteilen. Es wurden folgende Natriumsalze gefunden: Formiat, Acetat, Propionat, Oxalat, n-Butyrat, i-Butyrat, n--Valerat, i-Valerat, Lactat, Citrat, Succinat, Adipat und Malat gefunden, sowie einige weitere Spezies, die aus den jeweiligen Detektionsmethoden nicht eindeutig zugeordnet werden konnten. Weiterhin wurde als anorganisches Natriumsalz Na₂CO₃ in den Proben gefunden und der Gehalt durch Säuretitration bestimmt.

Von der flüssigen Schmelze unter dem flotierten Feststoff wurden ebenfalls Proben zur Analyse entnommen. Sie enthielten qualitativ die gleichen organischen Salze aber in anderer quantitativer Zusammensetzung.

Die Restschmelze wurde sodann ausgegossen und erstarren lassen. Dabei kristallisierten in verschiedenen Abkühlphasen bei unterschiedlichen Temperaturen verschiedene feste Phasen mit unterschiedlicher Zusammensetzung aus.

Die nachstehende Tabelle zeigt den Anteil des jeweiligen Na-Salzes in der Gesamtmenge d.h. inklusive der gemessenen Mengen in der Restschmelze sowie die Ausbeute als Verhältnis des in den Produkten wiedergefundenen Kohlenstoffs (C-Salze) zu dem Gesamtkohlenstoff im Input-Glycerin (C-Glycerin).

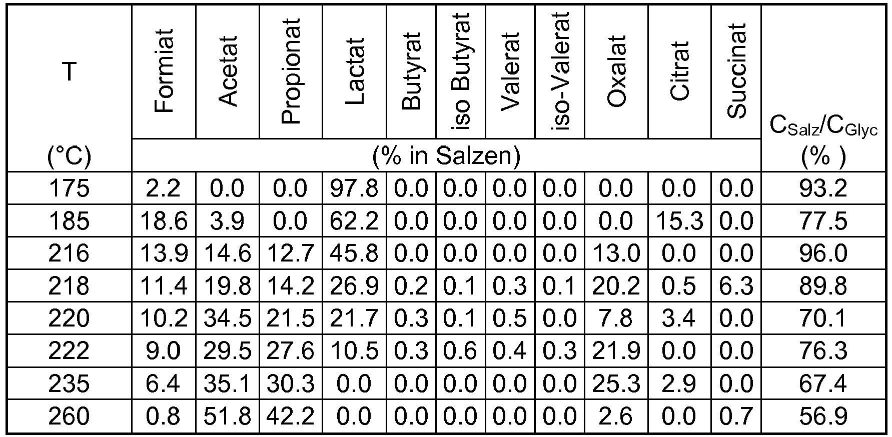

Es ist klar ersichtlich, dass die Salzanteile mit der Temperatur variieren, z.B.:
- Der Acetatanteil nimmt innerhalb des Intervalls 175 - 260 °C von 0 Gew% auf rund 52 Gew% zu.
- Der Lactatanteil nimmt innerhalb des Intervalls 175 - 260 °C von rund 98 Gew% auf 0 Gew% ab
- Der Formiatanteil ist zwischen 180 und 200 °C maximal mit ca. 19%

Somit kann die Reaktionstemperatur als Steuerungsparameter für die Selektivität eingesetzt werden, je nachdem, welches Salz bevorzugt gewonnen werden soll.

Zusätzlich kann die unterschiedliche Verteilung der Salze in Feststoff und Schmelze zur selektiven Gewinnung ausgenutzt werden.

Außerdem können verschiedene Salze gezielt in höherer Reinheit gewonnen werden durch kontrollierte (fraktionierte) Kristallisation der Restschmelze.

Die Ausbeuten an organischen Natriumsalzen (letzte Spalte) nehmen mit zunehmender Temperatur ab. Gleichzeitig nehmen die Ausbeuten an Na-Carbonat zu. Der zu 100% fehlende Kohlenstoffanteil aus der letzten Spalte wird überwiegend durch Na-Carbonat abgedeckt.

### Beispiel 4:

Der Reaktor wurde analog Beispiel 3 mit 20 kg Wasser und 60 kg fester NaOH beschickt, entsprechend einer NaOH-Konzentration von 75 Gew%. Der Liquidusbereich für diese Konzentration beträgt ca. 124 °C (zwischen Schmelzpunkt 71 °C und Siedepunkt 195 °C )

Die Temperatur wurde auf einen Wert von 180 °C eingestellt, d.h. 15°C unterhalb der Siedetemperatur.

Nach Inertisieren mit Stickstoff wurde auf 80 °C vorgeheiztes reines Glycerin (99.6%) mit einer Rate von 14 kg/h zudosiert, insgesamt 32.5 kg.

Die Reaktion lief ca. 1 ¼ Stunde bei konstant 180 °C ab, danach begann trotz beibehaltenem Regelsollwert die reale Temperatur der Reaktionsmischung langsam abzusinken. Dies könnte dadurch zu erklären sein, dass sich die Reaktionsmischung durch Bildung von Reaktionswasser langsam verdünnt (z.B. C₃H₈O₃ + NaOH --->CH₃CH(OH)COONa + H₂O + H₂), die Konzentration der Reaktionsmischung sich zunächst bei konstanter Temperatur bis zur Siedelinie verschiebt und danach bei weiterer Verdünnung die Siedetemperatur ( = theoretische Maximaltemperatur) des Systems absinkt.

Einem weiteren Absinken der Reaktionstemperatur wurde durch kontrolliertes Abziehen von Kondenswasser (ca. 16 g/min) aus der Wasserdampfphase entgegengewirkt. Das System wurde so für die restliche Reaktionszeit konstant an seinem Siedepunkt bei 173 °C gehalten, entsprechend einer Nominalkonzentration laut Literatur von ca. 66-67 Gew% NaOH

Die weitere Vorgehensweise und Beobachtungen entsprechen Beispiel 3, wobei im vorliegenden Fall eine wesentlich längere Nachreaktion beobachtet wurde, d.h. die Gasbildung war auch nach 8 Stunden mit ca. 9000 Litern Gesamtvolumen und 99.8% Wasserstoffanteil noch nicht ganz beendet.

Das feste Flotationsprodukt enthält Lactat, Oxalat, Formiat und Acetat im Verhältnis 51:25:12:1.

Die Restschmelze enthält kein Oxalat sondern nur Lactat, Formiat und Acetat im Verhältnis 54:11:1

Die nachstehende Tabelle zeigt die Gesamtausbeute bezogen auf den Inputstoff Glycerin:

| **Natriumsalz** | **% C_{salz} /C_{glycerin}** |
|---|---|
| Formiat | 7.8 |
| Acetat | 1.2 |
| Oxalat | 1.8 |
| Lactat | 67.8 |

Der Test wurde wahrscheinlich beendet, bevor die Reaktionen vollständig abgeschlossen waren, da sich insgesamt nur ca. 80% des Glycerins umgesetzt haben.

Vermutlich liegt die geringere Umsetzung im Vergleich zu anderen Tests zum einen an der hohen Zudosierungsrate und zum anderen auch daran, dass die Umsetzung während der Zudosierung nicht bei der maximal möglichen Systemtemperatur (= Siedetemperatur) und damit auch mit geringerer Reaktionsrate erfolgte.

## Patentansprüche

1. Verfahren zur Umsetzung von Glycerin wobei das Glycerin in einer wässrigen, flüssigen Lösung von Me-Hydroxid, wobei die Konzentration des Me-Hydroxids größer als die Sättigungskonzentration bei Raumtemperatur ist, bei Temperaturen über 150°C umgesetzt wird, wobei Me ausgewählt ist aus der Gruppe bestehend aus Alkali- und Erdalkalimetallen und deren Mischungen und mindestens ein Salz ausgewählt aus der Gruppe bestehend aus Formiat, Acetat, Propionat, Oxalat, Lactat, Butyrat, Valerat, Citrat, Adipat, Succinat, Malat und Carbonat sowie deren Mischungen und Wasserstoffgas (H₂) oder Methan (CH₄) sowie deren Mischungen entstehen.

2. Verfahren zur Umsetzung von Glycerin nach Anspruch 1, **dadurch gekennzeichnet, dass** das Verfahren bei einem Druck von 800 bis 1500 hPa ausgeführt wird.

3. Verfahren zur Umsetzung von Glycerin nach einem der Ansprüche 1 bis 2, **dadurch gekennzeichnet, dass** bei dem Verfahren NaOH oder KOH sowie deren Mischungen als Me-Hydroxid eingesetzt wird.

4. Verfahren zur Umsetzung von Glycerin nach einem der Ansprüche 1 bis 2, **dadurch gekennzeichnet, dass** bei dem Verfahren NaOH als Me-Hydroxid eingesetzt wird.

5. Verfahren zur Umsetzung von Glycerin nach einem der Ansprüche 1 bis 4, **dadurch gekennzeichnet, dass** die Konzentration von Me-Hydroxid größer als 54 Gew% ist.

6. Verfahren zur Umsetzung von Glycerin nach einem der Ansprüche 1 bis 5, **dadurch gekennzeichnet, dass** das Verfahren in einem Temperaturbereich innerhalb von 15°C um den Siedepunkt der Me-Hydroxid-Lösung ausgeführt wird.

7. Verfahren zur Umsetzung von Glycerin nach einem der Ansprüche 1 bis 6, **dadurch gekennzeichnet, dass** das Verfahren bei Temperaturen über 160°C ausgeführt wird und hauptsächlich Lactat erzeugt wird.

8. Verfahren zur Umsetzung von Glycerin nach einem der Ansprüche 1 bis 6, **dadurch gekennzeichnet, dass** das Verfahren bei Temperaturen zwischen 230-260°C ausgeführt wird und hauptsächlich Acetat, Propionat und Oxalat erzeugt wird.

9. Verfahren zur Umsetzung von Glycerin nach einem der Ansprüche 1 bis 8, wobei die wässerige Lösung von Me-Hydroxid unter ständigem Umrühren bei Temperaturen an dem Siedepunkt gehalten wird und das Glycerin in die Me-Hydroxid Lösung zudosiert wird.

10. Verfahren zur Umsetzung von Glycerin nach Anspruch 9, wobei das Glycerin vorgewärmt wird, bevor es in die Me-Hydroxid Lösung zu dosiert wird.

11. Verfahren zur Umsetzung von Glycerin nach einem der Ansprüche 1 bis 10, **dadurch gekennzeichnet, dass** das verwendete Glycerin aus der Herstellung von Biodiesel stammt.

12. Verfahren zur Umsetzung von Glycerin nach einem der Ansprüche 1 bis 11, **dadurch gekennzeichnet, dass** das Verfahren bei Rückflusskühlung ausgeführt wird.

13. Verfahren zur Umsetzung von Glycerin nach einem der Ansprüche 1 bis 12, wobei es sich um ein kontinuierliches Verfahren handelt.

14. Verfahren zur Umsetzung von Glycerin nach einem der Ansprüche 1 bis 12, wobei es sich um ein Batch-Verfahren handelt.

15. Verfahren zur Umsetzung von Glycerin nach einem der Ansprüche 1 bis 14, wobei dem Verfahren das durch die Umsetzung von Glycerin entstehende Wasser ganz oder zum Teil entzogen wird.

## Claims

1. A process for reacting glycerin wherein the glycerin is reacted at temperatures above 150°C in an aqueous, liquid solution of Me hydroxide, wherein the concentration of the Me hydroxide is greater than the saturation concentration at room temperature, wherein Me is selected from the group consisting of alkali metals and alkaline earth metals and mixtures thereof, and said reaction affords at least one salt selected from the group consisting of formate, acetate, propionate, oxalate, lactate, butyrate, valerate, citrate, adipate, succinate, malate and carbonate and mixtures thereof and hydrogen gas (H₂) or methane (CH₄) and mixtures thereof.

2. The process for reacting glycerin according to claim 1, wherein said process is carried out under a pressure of 800 to 1500 hPa.

3. The process for reacting glycerin according to one of claims 1 to 2, wherein NaOH or KOH as well as mixtures thereof are employed as the Me hydroxide in the process.

4. The process for reacting glycerin according to one of claims 1 to 2, wherein NaOH is employed as the Me hydroxide in the process.

5. The process for reacting glycerin according to one of claims 1 to 4, wherein the concentration of Me hydroxide is greater than 54 wt.

6. The process for reacting glycerin according to one of claims 1 to 5, wherein the process is carried out in a temperature range within 15 °C, of the boiling point of the Me hydroxide solution.

7. The process for reacting glycerin according to one of claims 1 to 6, wherein the process is carried out at temperatures above 160 °C and lactate is principally produced.

8. The process for reacting glycerin according to one of claims 1 to 6, wherein the process is carried out at temperatures between 230-260 °C, and acetate, propionate and oxalate are principally produced.

9. The process for reacting glycerin according to one of claims 1 to 8, wherein the aqueous solution of Me hydroxide is maintained with continuous stirring at temperatures around the boiling point and the glycerin is metered the Me hydroxide solution.

10. The process for reacting glycerin according to claim 9, wherein the glycerin is preheated before being metered into the Me hydroxide solution.

11. The process for reacting glycerin according to one of claims 1 to 10, wherein the glycerin used originates from the production of biodiesel.

12. The process for reacting glycerin according to one of claims 1 to 11, wherein the process is carried out with reflux cooling.

13. The process for reacting glycerin according to one of claims 1 to 12, wherein said process is a continuous process.

14. The process for reacting glycerin according to one of claims 1 to 12, wherein said process is a batch process.

15. The process for reacting glycerin according to one of claims 1 to 14, wherein the water produced by the reaction of glycerin in the process is totally or partially removed.

## Revendications

1. Procédé de transformation du glycérol, le glycérol étant transformé à des température supérieures à 150 °C dans une solution liquide aqueuse d'un hydroxyde de Me, la concentration dudit hydroxyde de Me étant supérieure à la concentration de saturation à température ambiante, Me étant choisi dans le groupe constitué de métaux alcalins et alcalino-terreux et de leurs mélanges, ladite transformation permettant d'obtenir au moins un sel choisi dans le groupe constitué de formate, acétate, proprionate, oxalate, lactate, butyrate, valérate, citrate, adipate, succinate, malate et de carbonate et de leurs mélanges, ainsi que de l'hydrogène gazeux (H₂) ou du méthane (CH₄) et leurs mélanges.

2. Procédé de transformation de glycérol selon la revendication 1, **caractérisé en ce que** ledit procédé est mis en oeuvre à une pression comprise entre 800 et 1500 hPa.

3. Procédé de transformation de glycérol selon l'une des revendications 1 à 2, **caractérisé en ce que** lors dudit procédé on met en oeuvre, en tant qu'hydroxyde de Me, du NaOH ou du KOH ainsi que leurs mélanges.

4. Procédé de transformation de glycérol selon l'une des revendications 1 à 2, **caractérisé en ce que** lors dudit procédé on met en oeuvre, en tant qu'hydroxyde de Me, du NaOH.

5. Procédé de transformation de glycérol selon l'une des revendications 1 à 4, **caractérisé en ce que** la concentration d'hydroxyde de Me est supérieure à 54 % en poids.

6. Procédé de transformation de glycérol selon l'une des revendications 1 à 5, **caractérisé en ce que** ledit procédé est mis en oeuvre dans une plage de températures pouvant dévier du point d'ébullition de la solution d'hydroxyde de Me de 15 °C vers le bas ou vers le haut.

7. Procédé de transformation de glycérol selon l'une des revendications 1 à 6, **caractérisé en ce que** ledit procédé est mis en oeuvre à des températures supérieures à 160 °C et permet d'obtenir essentiellement du lactate.

8. Procédé de transformation de glycérol selon l'une des revendications 1 à 6, **caractérisé en ce que** ledit procédé est mis en oeuvre à des températures entre 230 et 260 °C et permet d'obtenir essentiellement de l'acétate, du proprionate et de l'oxalate.

9. Procédé de transformation de glycérol selon l'une des revendications 1 à 8, la solution aqueuse d'hydroxyde de Me étant maintenue sous agitation à des températures correspondant audit point d'ébullition et ledit glycérol étant ajouté à la solution d'hydroxyde de Me de manière contrôlée.

10. Procédé de transformation de glycérol selon la revendication 9, ledit glycérol étant préchauffé avant d'être ajouté à la solution d'hydroxyde de Me de manière contrôlée.

11. Procédé de transformation de glycérol selon l'une des revendications 1 à 10, **caractérisé en ce que** le glycérol mis en oeuvre est issu de la production de biodiesel.

12. Procédé de transformation de glycérol selon l'une des revendications 1 à 11, **caractérisé en ce que** ledit procédé est mis en oeuvre en chauffant à reflux.

13. Procédé de transformation de glycérol selon l'une des revendications 1 à 12, s'agissant d'un procédé continu.

14. Procédé de transformation de glycérol selon l'une des revendications 1 à 12, s'agissant d'un procédé discontinu.

15. Procédé de transformation de glycérol selon l'une des revendications 1 à 14, l'eau produite par ladite transformation de glycérol étant partiellement ou en totalité retirée dudit procédé.
